# EUROPEAN PATENT APPLICATION

(11) **EP 4 623 866 A1**
(43) Date of publication of application: **01.10.2025**
(21) Application number: 24222508.4
(22) Date of filing: 20.12.2024
(51) Int. Cl.: A61F 2/24, A61F 2/95

(54) **SUTURE CATHETER TENSIONER FOR DELIVERY SYSTEM**

(30) Priority: 28.03.2024 US 202463571113 P
(71) Applicant: Cephea Valve Technologies, Inc., Abbott Park, Illinois 60064 (US)
(72) Inventor: HAKE, Jarred, Fremont, CA 94536 (US); CARLSON, Chase, San Jose, CA 95218 (US)
(74) Representative: LKGLOBAL Lorenz & Kopf Patentanwalt Attorney at Law PartG mbB

(57) **Abstract**

A system, having a delivery assembly having a handle assembly and a catheter assembly, the handle assembly having a control mechanism configured to control a suture catheter of the catheter assembly, the control mechanism having a puck configured to translate axially relative to the handle assembly in order to correspondingly axially translate the suture catheter, the control mechanism having a spring configured to exert at least a minimum load to proximally bias the suture catheter; and a prosthetic heart valve having a plurality of pins configured to engage with one or more tethers indirectly coupled to the suture catheter.

## Description

### CROSS-REFERENCE TO RELATED APPLICATIONS

The present application claims priority to U.S. Provisional Serial No. 63/571,113, filed March 28, 2024.

### BACKGROUND

The present disclosure relates generally to devices, systems and methods for delivering an interventional device into a patient for implantation.

Intravascular medical procedures allow the performance of therapeutic treatments in a variety of locations within a patient's body while requiring only relatively small access incisions. An intravascular procedure may, for example, eliminate the need for open-heart surgery, reducing risks, costs, and time associated with an open-heart procedure. The intravascular procedure also enables faster recovery times with lower associated costs and risks of complications. An example of an intravascular procedure that significantly reduces procedure and recovery time and cost over conventional open surgery is a heart valve replacement or repair procedure in which an artificial valve or valve repair device is guided to the heart through the patient's vasculature. For example, a catheter is inserted into the patient's vasculature and directed to the inferior vena cava. The catheter is then urged through the inferior vena cava toward the heart by applying force longitudinally (e.g., pushed forward) to the catheter. Upon entering the heart from the inferior vena cava, the catheter enters the right atrium. The catheter may be guided across the atrial septum (e.g., via a guidewire that has already been passed through the atrial septum) into the left atrium. The distal end of the catheter may be deflected by one or more deflecting mechanisms in order to align the distal end of the catheter, as well as a medical device positioned therein, with the mitral valve. Catheter deflection can be achieved by tension cables, or other mechanisms positioned inside the catheter. Precise control of the distal end of the catheter allows for more reliable and faster positioning of a medical device and/or implant and other improvements in the procedures.

### BRIEF SUMMARY

One aspect of the disclosure provides a system, comprising: a delivery device having a handle assembly and a catheter assembly, the handle assembly having a control mechanism with a spring, and the catheter assembly including a suture catheter operably coupled to the control mechanism such that axial translation of the control mechanism relative to the handle assembly correspondingly axially translates the suture catheter relative to the handle assembly; a suture rigging assembly having a plurality of suture tethers; and a prosthetic heart valve, wherein in an assembled condition of the system, the suture rigging assembly is coupled to the suture catheter, the plurality of suture tethers are coupled to the prosthetic heart valve, and the spring exerts a load to proximally bias the suture catheter relative to the control mechanism to maintain tension on the plurality of suture tethers.

In one example, the control mechanism further comprises: a translating bore nut having a catheter connection interface that connects to the suture catheter, the translating bore nut having a distal portion; the spring extending around an outer circumference of the distal portion of the translating bore nut, a proximal end of the spring pressing upon an external shoulder of the translating bore nut and exerting the load.

In one example, the control mechanism further comprises: a spring bar having an internal shoulder, a distal end of the spring pressing against the internal shoulder of the spring bar.

In one example, the control mechanism further comprises: a bearing plate received within the control mechanism; and a static piston coupled to the bearing plate.

In one example, the static piston includes at least one sealing component arranged circumferentially with respect to a substantially cylindrical portion of the static piston such that a seal is maintained between the static piston and the translating bore nut.

In one example, the translating bore nut translates axially relative to the static piston, where a length of a proximal portion of the translating bore nut is larger than a total axial travel distance of the translating bore nut such that the seal is maintained between the translating bore nut and the static piston.

In one example, the spring bar defines a plurality of legs and a bore nut guide, with a proximal portion of the translating bore nut being disposed between the plurality of legs and the distal portion of the translating bore nut being received within the bore nut guide.

In one example, the spring bar includes a plurality of tension indicators configured to align with a distal end of the translating bore nut in order to indicate a tension level of the plurality of suture tethers.

In one example, the plurality of tension indicators are disposed on at least one leg of the spring bar.

In one example, the bearing plate defines a longitudinal slot configured to receive a base portion of the static piston.

In one example, the translating bore nut has a proximal portion defining an internal recess configured to receive a substantially cylindrical portion of the static piston.

In one example, the control mechanism further comprises an actuator configured to allow for the axial translation of the control mechanism relative to the handle assembly.

In one example, the suture catheter is configured to be advanced distally to engage with a coupling ring of the suture rigging assembly.

In one example, upon coupling of the suture catheter and coupling ring, the suture catheter is retracted proximally to load the prosthetic heart valve in a valve cover of the catheter assembly.

In one example, in the assembled condition of the system, the suture catheter is configured to be advanced distally by distally translating the control mechanism relative to the handle assembly to disconnect the plurality of suture tethers from the prosthetic heart valve to allow for deployment of the prosthetic heart valve into a native valve.

Another aspect of the disclosure provides a method of loading a prosthetic heart valve, comprising: distally advancing a suture catheter relative to a handle assembly; coupling the suture catheter with a suture rigging assembly, the suture rigging assembly having a plurality of suture tethers being coupled to the prosthetic heart valve in an assembled state; proximally retracting the suture catheter relative to the handle assembly while a spring exerts a load to proximally bias the suture catheter relative to the control mechanism so as to maintain tension on the plurality of suture catheters.

In one example, distally advancing a control mechanism relative to the handle assembly of a delivery device causes distal advancement of the suture catheter relative to the handle assembly.

In one example, proximally retracting the control mechanism relative to the handle assembly of the delivery device causes proximal retraction of the suture catheter relative to the handle assembly.

In one example, the suture catheter is proximally retracted based upon one or more tension indicators indicative of a tension on the plurality of suture catheters.

In one example, the one or more tension indicators include at least a proximal tension indicator and a distal tension indicator such that alignment of a translating bore nut of the control mechanism with the proximal indicator is indicative of low tension.

### BRIEF DESCRIPTION OF THE DRAWINGS

A more complete appreciation of the subject matter of the present disclosure and the various advantages thereof can be realized by reference to the following detailed description in which reference is made to the accompanying drawings in which:
FIG. 1 is a perspective view of a delivery system configured for delivering, positioning and deploying a prosthetic heart valve, including a handle assembly and a catheter assembly;
FIG. 2 is an exploded view of a portion of the handle assembly of the delivery system of Fig. 1, including valve cover retraction knob and cone assembly;
FIG. 3 is a side view of a portion of the catheter assembly of the delivery system of FIG. 1, including a catheter shaft, coiled and/or braided section, valve cover, and nosecone;
FIG. 4A is a transverse cross-sectional view of the catheter shaft of FIG. 3, showing various components thereof;
FIGS. 4B-4C are transverse cross-sectional views of a catheter assembly, operable with the delivery system of Fig. 1, according to another aspect of the disclosure;
FIG. 5A is a longitudinal cross-section of the catheter assembly of FIG. 3, showing the nested arrangement of various components thereof;
FIG. 5B is a longitudinal cross-section of a catheter assembly, operable with the delivery system of FIG. 1 and according to another aspect of the disclosure, showing the nested arrangement of various components thereof;
FIG. 6 is a perspective view of the suture rigging assembly showing radiopaque markers attached to the tethers;
FIG. 7 is a perspective view of the suture rigging assembly attached between the delivery catheter and a prosthetic heart valve;
FIG. 8 is a perspective view of a control mechanism according to one or more aspects of the disclosure;
FIG. 9 is an exploded view of the control mechanism of FIG. 8;
FIGS. 10A-B are cross sectional views of the control mechanism of Fig. 8 in different states;
FIG. 11 is a perspective view of the control mechanism of FIG. 8 showing indicator markings according to one or more aspects of the disclosure; and
FIGS. 12A-B are fluoroscopic views of an initial stage of valve deployment according to one or more aspects of the disclosure.

### DETAILED DESCRIPTION

As used herein, the term "inflow end," when used in connection with a prosthetic heart valve, refers to the end of the heart valve through which blood enters when the heart valve is functioning as intended, whereas the term "outflow end," when used in connection with a prosthetic heart valve, refers to the end of the heart valve through which blood exits when the heart valve is functioning as intended. For a prosthetic mitral valve, the inflow end is closest to the left atrium when the heart valve is implanted in a patient, and the outflow end is closest to the left ventricle when the heart valve is implanted in a patient. Further, when used herein in connection with a delivery device, the terms "proximal" and "distal" are to be taken as relative to a user operating the device in an intended manner. "Proximal" is to be understood as relatively close to the user and "distal" is to be understood as relatively farther away from the user. Also as used herein, the terms "substantially," "generally," and "about" are intended to mean that slight deviations from absolute are included within the scope of the term so modified.

Native mitral and tricuspid valves, especially the native tricuspid valve, are typically larger in diameter than a native aortic valve. Thus, collapsible and expandable prosthetic mitral and tricuspid valves are typically much larger in diameter than collapsible and expandable prosthetic aortic and pulmonary valves. As a result, transcatheter delivery devices for prosthetic mitral and tricuspid valves are typically larger in size than transcatheter delivery devices for prosthetic aortic and pulmonary valves. It is generally desirable for transcatheter delivery devices to be as small as possible while still being capable of retaining the prosthetic heart valve therein and otherwise functioning as intended.

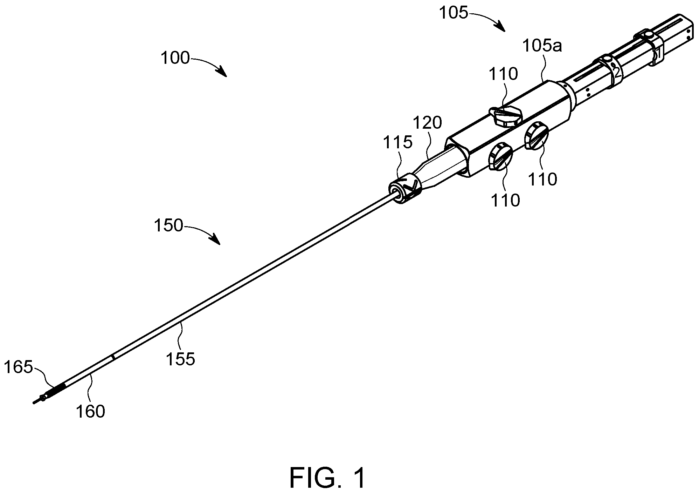

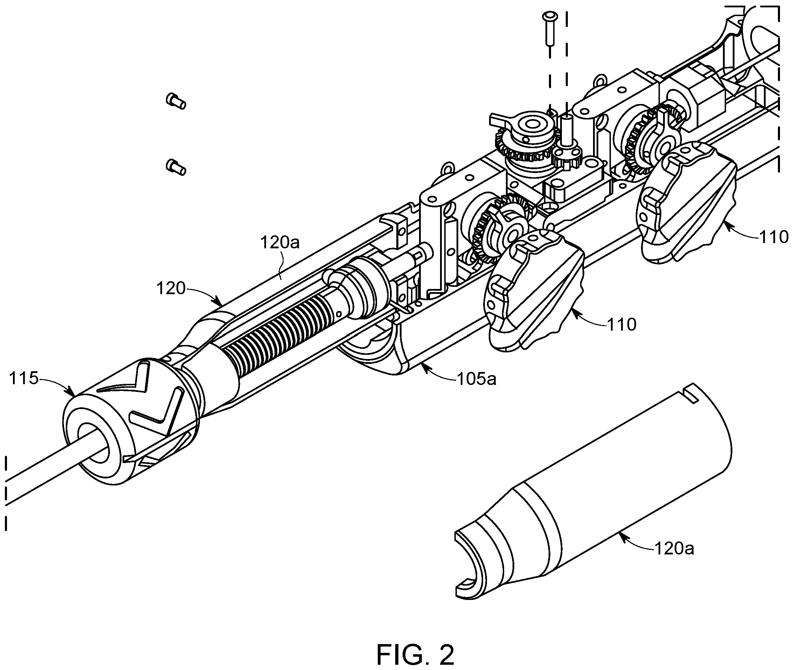

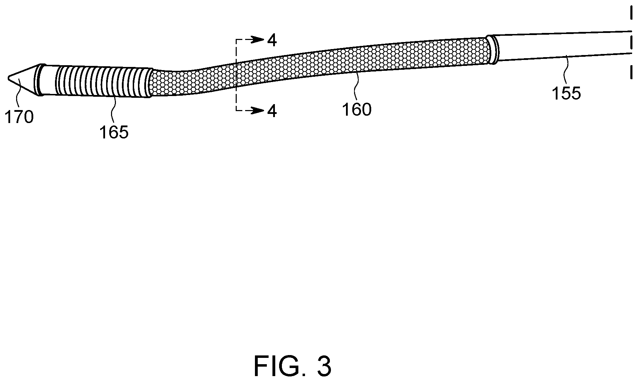

FIG. 1 is a perspective view of a delivery system 100 configured for delivering, positioning and deploying a prosthetic heart valve, including a handle assembly 105 and a catheter assembly 150. FIG. 2 is an exploded view of a portion of the handle assembly 105 of the delivery system 100 of FIG. 1, including valve cover retraction knob 115 and cone assembly 120 (which may also be referred to as a locking assembly herein). FIG. 3 is a side view of a distal end portion of the catheter assembly 150 of the delivery system 100 of FIG. 1, including a catheter shaft 155, coiled and/or braided section 160, valve cover 165 (which may also be referred to as a valve capsule), and an atraumatic distal tip or nosecone 170.

Delivery system 100 generally includes a handle assembly 105 and a catheter assembly 150. Catheter assembly 150 extends from a proximal end coupled to handle assembly 105 to an atraumatic tip or nosecone (not shown in FIG. 1) at a distal end and includes a plurality of catheter and/or hypotube components, at least some of which are longitudinally slidable relative to one another and provide different functionality during operation of delivery system 100 to enable effective delivery and deployment of a prosthetic heart valve, such as a prosthetic mitral valve. While the catheter assembly 150 is depicted as having a length relative to handle assembly 105, it should be understood that the catheter assembly 150 can have any suitable length and, in some examples, has a much greater length than the handle assembly 105.

Attached to a housing 105a of the handle assembly 105 can be various control mechanisms for controlling one or more aspects of the delivery. For example, attached to the housing 105a of the handle assembly 105 can be one or more knobs 110 for controlling steering of the of the catheter assembly 150. Additional details regarding steering operations that may be utilized in conjunction with the components and features described herein are described in U.S. Patent Application Publication No. 2023/0364387, filed April 19, 2023, entitled "Advanced 3-Way Steering." The handle assembly 105 can also include a valve cover retraction knob 115 and a locking or cone assembly 120. The valve cover retraction knob 115 can be rotated to allow for retraction of valve cover 165 during delivery and/or expression of a prosthetic heart valve. In some examples, rotating valve cover retraction knob 115 results in proximal translation of a catheter member that is attached to the valve cover 165, allowing for the valve cover 165 to uncover a self-expandable prosthetic heart valve to allow the prosthetic heart valve to self-expand. The cone assembly 120 can be rotated relative to housing 105a, as is explained in greater detail below, to lock or unlock an outer catheter relative to an inner catheter.

The catheter assembly 150 can include a catheter shaft 155, a coiled and/or braided section 160, a valve cover 165, and a nosecone (not shown in FIG. 1), as will be described in greater detail below. It should also be understood that catheter assembly 150 may include a number of additional catheter members nested within catheter shaft 155, as described in greater detail below.

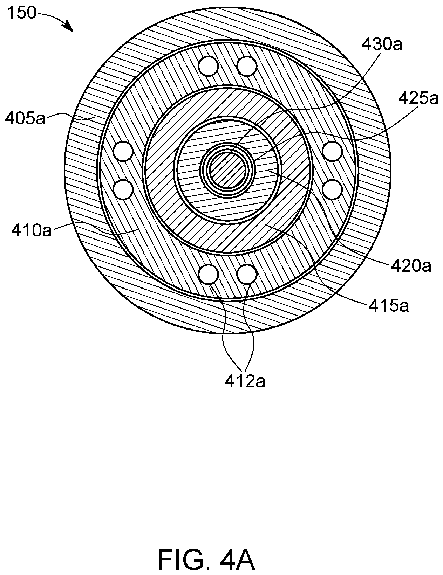

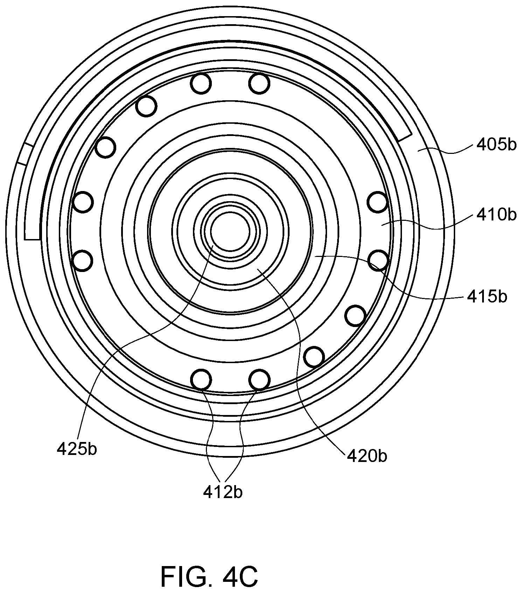

FIG. 4A is a transverse cross-sectional view of the catheter shaft 155 of FG. 3, showing various components thereof, including addition catheter components nested therein.

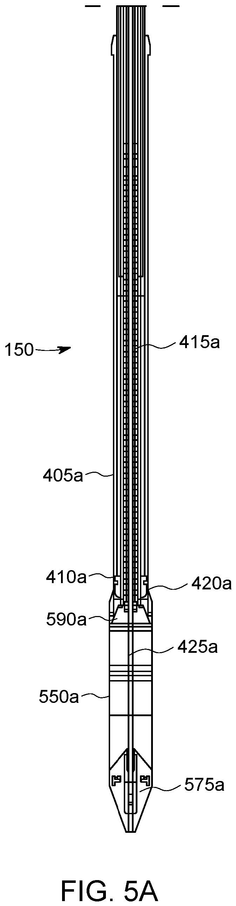

As shown in FIG. 4A, which is a transverse cross-sectional view of catheter assembly 150 taken along line 4-4 of FIG. 3, these components may include an outer sheath 405a, a steering catheter 410a, an extension catheter 415a, a suture catheter 420a, and a nosecone catheter 425a, all arranged in a concentric nested relationship. The arrangement of these components, as well as valve cover 550a, nosecone 575a, and valve retainer 590a (also referred to as "can") is shown in the longitudinal cross-section of catheter assembly 150 shown in FIG. 5A. During delivery, the proximal petals of the compressed prosthetic valve can be nested inside of the valve retainer when the prosthetic valve is held back by the suture catheter, which is under tension. In one example, valve retainer 590a may be attached directly to a tip ring of the steering catheter 410a. As illustrated in the figures, nosecone catheter 425a has a lumen sized to receive a guidewire 430a therein. Each of these components is described in detail below. It should be understood that the valve cover 165 of FIG. 1 and FIG. 3 may be substantially the same as or identical to the other valve covers described herein. Similarly, it should be understood that the atraumatic distal tip or nosecone 170 of FIG. 3 may be substantially the same as or identical to the other atraumatic distal tips or nosecones described herein. In some examples, the suture catheter 420a may have a plurality of sutures or other wire loops attached to a distal end thereof, the suture loops or wire loops configured to selectively maintain a connection between the prosthetic heart valve and the delivery device.

To selectively control the curvature of a distal section of steering catheter 410a, the steering catheter 410a may be provided with a plurality of tension cables (not shown). The tension cables may travel from handle assembly 105 (e.g., one or more of the knobs 110) through a plurality of lumens 412a (which in some embodiments may be polymer tubes, such as Nylon, Pebax, Polyimide, or polytetrafluoroethylene or "PTFE" tubes, or any other type of polymer) to the distal end of steering catheter 410a. In one embodiment, steering catheter 410a may include four lumens 412a equally spaced at 90° intervals around the circumference of steering catheter 410a. In the embodiment of FIG. 4A, steering catheter 410a may include four pairs of lumens 412a (a total of eight lumens 412a), with the pairs of lumens equally spaced at 90° intervals around the circumference of steering catheter 410a. Any number of these lumens 412a (or pairs of lumens) may be provided depending on the various directions of deflection that may be desired. For example, six pairs of lumens 412a may be provided, with each pair of lumens 412a housing one steering cable of a pair of steering cables coupled to a corresponding steering knob 110 (*e.g.,* two steering cables attached to each steering knob 110, with each steering cable extending through one pair of lumens 412a, for a total of twelve lumens 412a arranged in six pairs).

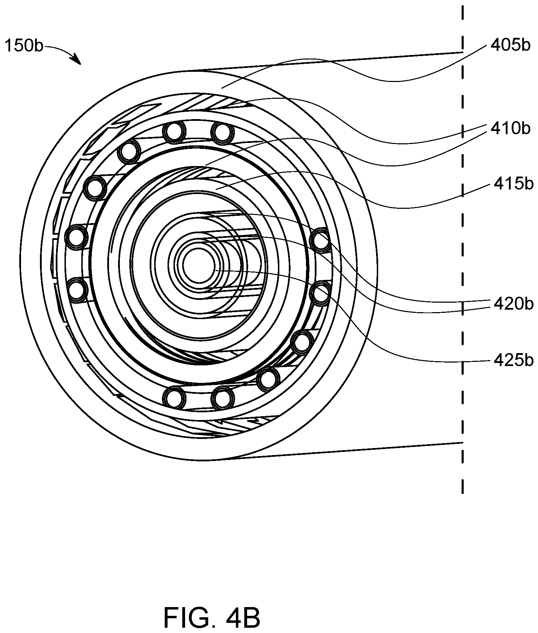

FIGS. 4B-4C are transverse cross-sectional views of a catheter assembly 150b, operable with the delivery system of FIG. 1 (*e.g.,* in place of catheter assembly 150), according to another aspect of the disclosure.

In this example, catheter assembly 150b omits an extension catheter (e.g., extension catheter 415a described above) and includes an outer catheter 405b, a steering catheter 410b, an intermediate catheter 415b, a suture catheter 420b, and a nosecone catheter 425b. Also in this example, the steering catheter 410b includes six pairs (a total of 12) of lumens 412b, which again may be formed with polymer tubes. In this example, the extension catheter is omitted and an intermediate catheter 415b is implemented in its place. Implementation of the intermediate catheter 415b can advantageously reduce an overall diameter (e.g., French size) of the catheter assembly 150. A distal portion of the intermediate catheter 415b can be fixed (e.g., by a threaded connection) to a distal tip of the steering catheter 410b. The distal portion of the intermediate catheter 415b can have a portion of greater diameter, while the proximal portion can be adjacent to the suture catheter 420b to allow for advancement/retraction of the suture catheter 420b during deployment.

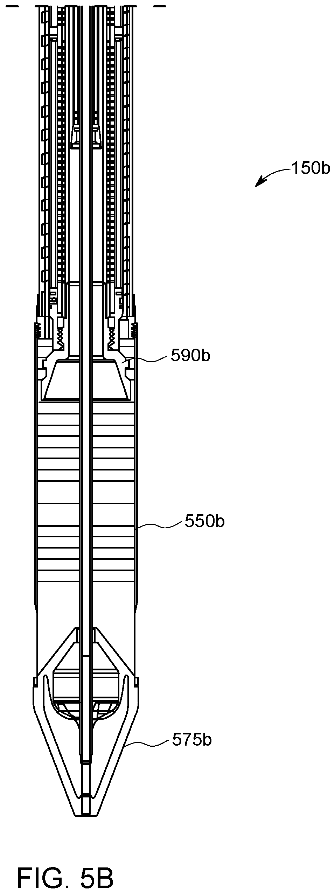

The arrangement of these components, as well as valve cover 550b, nosecone 575b, and valve retainer 590b is shown in the longitudinal cross-section of catheter assembly 150b shown in FIG. 5B.

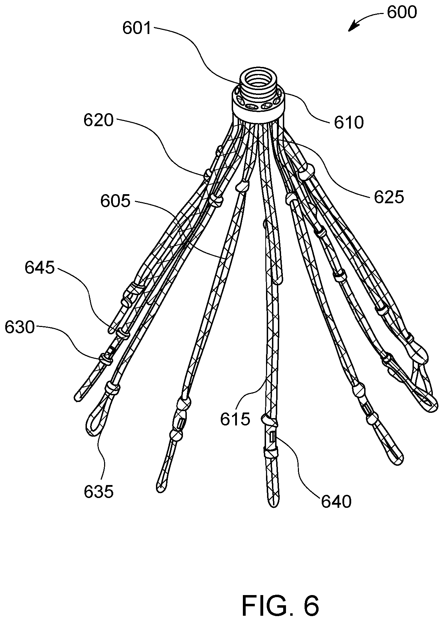

A suture rigging assembly 600 that assists in collapsing and drawing prosthetic heart valve V into delivery device 100 is shown in FIGS. 6-7. Suture rigging assembly 600 includes a coupling ring 601 to which a plurality of suture tethers may be attached. An exemplary suture rigging assembly and coupling ring is shown in U.S. Pre-Grant Publication 2023/0030110, filed July 28, 2022.

One or more suture threads 605 may be attached to the head of coupling ring 601. The suture threads 605 may be comprised of various materials, both man-made and natural. Examples of natural suture materials may include, but are not limited to, silk, linen, and catgut. Examples of synthetic suture materials may include, but are not limited to, textiles such as nylon or polyester, or flexible metallic cables. An elongated suture thread 605 may be threaded through a plurality of the bores 610 in coupling ring 601 to form tethers 615. Suture rigging assembly 600 may include a coupling ring 601 having at least one tether 615 or a plurality of tethers. For example, suture thread 605 may be threaded distally through a bore 610 in inner ring of coupling ring 601 and then proximally though an adjacent bore in outer ring of coupling ring 601, thereby forming an elongated loop or tether 615 extending distally from coupling ring 601. Thus, tether 605 includes two lengths of suture thread extending side-by-side and continuous with one another at their distal ends. Suture thread 605 may then be threaded distally through an adjacent bore 610 in inner ring and then proximally through an adjacent bore in outer ring, thereby forming another elongated loop or tether 615 extending distally from coupling ring 601. This pattern may be repeated to form a plurality of elongated loops or tethers 615 around the entire circumference of coupling ring 601. Thus, tethers 615 may be formed by a single continuous suture thread 605, with the leading and trailing ends of the suture thread being joined to one another by one or more terminating knots.

Suture threads forming a tether 615 may be joined together by a first knot or stop knot 620 at a spaced distance from coupling ring 601. Stop knots 620 reduce the ability of suture threads to separate too far from one another or to create a large loop or lasso. The distance between the knots on a tether 615 will define the maximum loop or lasso that can be formed by the tether. As a result of using knots, any loop or lasso able to form will be smaller in size than the loop or lasso in a tether 615 that does not have any knots. Preventing the formation of large loops or lassos is important because a large loop or lasso may become entangled with the apexes of the ventricular anchor of a prosthetic heart valve, thereby impairing the user's ability to pull back the entangled tether 615 after valve has been deployed. As shown in FIG. 6, stop knots 620 in adjacent tethers 615 preferably are formed at different spaced distances from coupling ring 601. For example, the stop knots 620 in a first group of tethers 615 may be spaced a first distance from coupling ring 601, and the stop knots in a second group of tethers that alternate with the tethers in the first group may be spaced a second distance greater than the first distance from the coupling ring. By offsetting the stop knots 620 in adjacent tethers 615 from one another, the tethers are better able to collapse to a smaller, more compact cross-sectional size within the confines of delivery device 100.

The stop knots 620 in tethers 615 create in each tether an upper or proximal connecting loop 625 between the knot and coupling ring 601. The ability of suture thread 605 to move freely within bores 610 enables the lengths of tethers 615 to self-adjust to a certain degree. That is, each tether 615 is free to move proximally until its stop knot 620 contacts coupling ring 601 and is free to move distally until the stop knots in the adjacent tethers contact the coupling ring. Therefore, as one tether 615 lengthens as it moves distally, there is a corresponding proximal movement and shortening of the adjacent tethers on either side of it, in the manner of a pulley. This adjustment in the lengths of tethers 615 enables a balancing of the load imparted to each of the tethers as prosthetic heart valve is collapsed during loading into delivery device 100 or during re-sheathing. For example, if a shorter tether 615 experiences a higher tensile stress upon the loading of prosthetic heart valve into delivery device 100, that tether may lengthen as the adjacent tethers shorten until the tensile stress on all of the tethers reaches an equilibrium point at which the total tensile stress is substantially evenly distributed among all of the tethers. Maintaining a balanced load among tethers 615 prevents any one of the tethers from becoming overloaded and breaking, which can impede the functionality of the entire system. Further, more evenly distributing the load among tethers 615 enables the overall tensile capacity of suture rigging assembly 600 to be increased.

Additional knots may also be formed at the distal or closed end of tethers 615. As shown in FIG. 6, a third knot or lower fixture knot 630 may be formed at a spaced distance from the distal end of tether 615, forming a closed attachment loop 635 therein. Attachment loops 635 are intended to hook onto the pins of prosthetic heart valve and to apply tension to assist in collapsing the prosthetic heart valve during loading into delivery device 100, as described more fully below. Preferably, attachment loops 635 have a relatively small size so that, following their release from pins during deployment, they do not become entangled with the pins or other structures of prosthetic heart valve, impeding proper deployment of the heart valve and removal of delivery device 100 from the patient.

To help visualize the locations of tethers 615, and in particular the positions of attachment loops 635, during the deployment of prosthetic heart valve in a patient, some embodiments of suture rigging assembly 600 may include a radiopaque marker 640 on all or at least some of the tethers. Radiopaque markers 640 may be formed of any material that can be readily visualized under fluoroscopy, including metals such as gold, platinum, platinum-iridium, tantalum, tantalum-tungsten, and others, and may take any shape. Preferably, radiopaque markers 640 have a bore or channel extending therethrough so that the markers may be threaded onto suture threads before lower fixture knot 630 is formed therein or as suture thread 605 is threaded through bores 610. In some embodiments, radiopaque markers 640 may be cylindrical, with a bore extending therethrough along the longitudinal axis of the cylinder. The radiopaque markers 640 provided on suture rigging assembly 600 need not all have the same shape, and different shapes may be assembled to various tethers 615 to indicate the orientation of prosthetic heart valve or to identify various portions thereof. Moreover, if any of tethers 615 is improperly affixed to prosthetic heart valve or becomes improperly affixed to the prosthetic heart valve during delivery of the heart valve into the patient or during deployment, radiopaque markers 640 may help to identify which of the tethers is improperly affixed and identify its location.

Radiopaque markers 640 may be held in a fixed position on tethers 615 by lower fixture knot 630 at the distal end of the marker and by a second or upper fixture knot 645 formed in the tether at the proximal end of the marker. Fixture knots 645 and 630 capture the radiopaque marker 640 therebetween and prevent it from sliding along the length of tether 615 toward or away from attachment loop 635. As a less preferable alternative, adhesives can be used to attach the radiopaque markers 640 at fixed positions to tethers 615. As a result, once a radiopaque marker 640 has been identified under fluoroscopy, the user will know the position of the attachment loop 635 associated with that marker.

The use of knots to form suture rigging assembly 600 provides several advantages. Firstly, it enables adhesives to be avoided, reducing sterilization, storage and biocompatibility issues that adhesives may create. The elimination of adhesives may also reduce the formation of very small particles during the use of delivery device 100, which particles could potentially be released into the patient's bloodstream. The use of knots throughout suture rigging assembly 600 also enables the assembly to be self-balancing, minimizing the tensile stress in any one tether 615 and increasing the overall tensile capacity of the suture rigging assembly. Finally, the various knots in each tether 615 keeps suture threads close to one another to prevent undesirable entanglement of the tethers with structures of prosthetic heart valve during deployment.

Suture rigging assembly 600 can be used to attach, load, and release a wide variety of heart valves to/from a wide variety of catheter-based delivery systems. Thus, suture rigging assembly 600 is designed to attach to a prosthetic heart valve and sustain a tensile load path between the heart valve and a delivery device as the heart valve is retracted into a sheath of the delivery device.

One way in which suture rigging assembly 600 may be used to collapse and load prosthetic heart valve V into the valve cover (e.g., valve cover 165) of delivery device 100 will now be described. Initially, suture rigging assembly 600 is attached to prosthetic heart valve V. This is accomplished by fitting some or, preferably, all of the attachment loops 635 at the distal ends of tethers 615 over respective pins on prosthetic heart valve V. Although this is described here as an initial step, it need not be the first step in the process. Suture rigging assembly 600 may be attached to delivery device 100 first, as described below, followed by the attachment of prosthetic heart valve V to the suture rigging assembly 600.

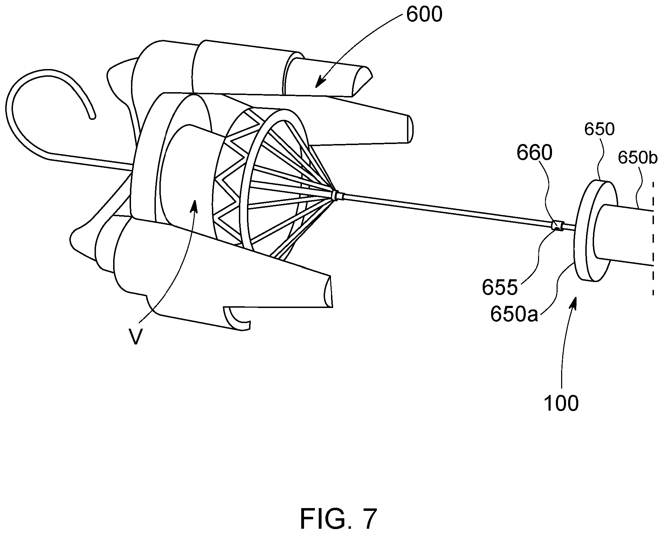

Referring to FIG. 7, a loading funnel 650 that can be used to help load prosthetic heart valve V into valve cover. In one embodiment, loading funnel 650 may include a funnel portion 650a located at its distal end and an elongated tubular portion 650b located at its proximal end. Funnel portion 650a may smoothly transition from a relatively large diameter at its distal end to a relatively small diameter where it meets tubular portion 650b. The diameter at the distal end of funnel portion 650a is preferably larger than the outer diameter of prosthetic heart valve V in its expanded condition, and the diameter of the funnel portion where it meets tubular portion 650b is preferably about the same as the diameter of the lumen of the tubular portion 650b. The outer diameter of tubular portion 650b is preferably slightly smaller than the inner diameter of valve cover and the length of the tubular portion may be about the same as the length of the valve cover, such that the tubular portion can be selectively inserted into and nest within the valve cover. Funnel portion 650a may include a plurality of slots (not shown) extending longitudinally along its inner surface. These slots are intended to accommodate the pins of prosthetic heart valve V and prevent them from bending laterally as the heart valve is being collapsed.

With the tubular portion 650b of loading funnel 650 coupled to a distal end of valve cover, controls located on the operating handle of delivery device 100 may be manipulated to cause suture catheter 655 to advance distally relative to the other components of the delivery device until the tip ring 660 of the suture catheter extends distally beyond the distal end of the tubular portion and into the interior of funnel portion 650a. At that point, the threads of coupling ring 601 may be threaded into the threaded portion of tip ring 660 at the distal end of suture catheter 655. FIG. 7 illustrates suture rigging assembly 600 connected to prosthetic heart valve V and aligned for connection to the suture catheter 655 of delivery device 100. Suture catheter 655 may then be retracted proximally, drawing suture rigging assembly 600 and prosthetic heart valve V proximally along with it. As proximal movement continues, tethers 615 are drawn into the lumen of the tubular portion 650b of loading funnel 650. This, in combination with the sloping walls of funnel portion 650a, causes the petals on atrial anchor of the prosthetic heart valve V to collapse toward the central axis of prosthetic heart valve V and, eventually, to enter the lumen of tubular portion 650b. Further proximal movement of suture catheter 655 continues until the petals on ventricular anchor also collapse toward the central axis of prosthetic heart valve V and the prosthetic heart valve is completely collapsed and completely positioned within the lumen of the tubular portion 650b. At that juncture, while maintaining tension on suture catheter 655, loading funnel 650 may be removed from valve cover, leaving the fully collapsed prosthetic heart valve V positioned completely within the valve cover. An atraumatic tip (not shown) of the delivery device may then be retracted to enclose the open distal end of valve cover.

In another embodiment, the loading funnel may have a generally cylindrical shape with internal threads at one end and an internal diameter that is about the same as the inner diameter of valve cover. The internal threads may mate with external threads at the free end of valve cover to join the loading funnel to the valve cover. A smooth radius on the lumen at the free end of the funnel may help to guide prosthetic heart valve V into the funnel lumen.

Once properly loaded, delivery device 100 may be inserted into a patient and directed to a target location, such as the mitral valve annulus, at which prosthetic heart valve V may be deployed. To deploy prosthetic heart valve V, valve cover is retracted proximally over valve V while the valve is maintained in position by extension catheter (or intermediate catheter). The ventricular anchor of valve V will then begin to expand until only the proximal end of the valve (i.e., atrial anchor) is held in a collapsed condition by the valve retainer (e.g., can) at the distal end of extension catheter. The accurate positioning and orientation of prosthetic heart valve V may then be confirmed, after which suture catheter 655 may be advanced distally, relieving tension in tethers 615 and allowing atrial anchor to escape from the valve retainer at the distal end of extension catheter and expand. Suture catheter 655 may be advanced further through the expanded prosthetic heart valve until tethers 615 slip off of pins. Suture catheter 655 may then be retracted back into outer delivery sheath, the atraumatic tip may be retracted to again close the open end of valve cover, and delivery device 100 may be removed from the patient.

During delivery of a prosthetic heart valve to a native valve, the delivery device can undergo complex bends and turns when advanced through the patient's vasculature and/or heart. For example, in transcatheter mitral valve replacement, a catheter assembly of a delivery system may be advanced through the inferior cava into the right atrium and turned toward the left atrium, passing through the septum. After passing through the septum, the catheter assembly may be deflected sharply, including in one or more planes, to concentrically align the catheter assembly with the native mitral valve. For catheter assemblies with nested catheters, such complex bends and turns can cause unintentional and/or undesirable relative movement of inner catheters relative to outer catheters. Such relative movement can result in slack (e.g., loss of tension and axial position) on the tethers that are coupled to the prosthetic heart valve.

As explained above, the suture catheter, via one of more tethers, maintains a tension on the prosthetic heart valve prior to delivery. In other words, as long as the suture tethers that have a first end connected to the suture catheter and a second end connected to the prosthetic heart valve remain tensioned, the prosthetic heart valve remains coupled to the delivery system and unable to prematurely deploy from the delivery system. Once the position and orientation of the prosthetic heart valve V are confirmed, tension in the tethers can be released by advancing the suture catheter distally (and thus advancing the suture tethers distally) relative to the prosthetic heart valve V until the suture tethers slip off of the pins of the prosthetic heart valve to which they were previously attached. Once the suture tethers disconnect from the prosthetic heart valve, it is free to escape the valve retainer and fully self-expand to an expanded state.

In the tensioned state of the suture tethers, the relative movement between catheters, in particular between the suture catheter and the outer catheter and/or steering catheter, can cause loss of tension in the tethers and axial (e.g., distal) movement of all or a portion of the prosthetic heart valve. Such loss of tension and/or distal movement is not desirable, as it can result in premature migration of the collapsed prosthetic heart valve from the valve cover. This can result in abrupt motion of the catheter during unsheathing the valve and unintended valve deployment position.

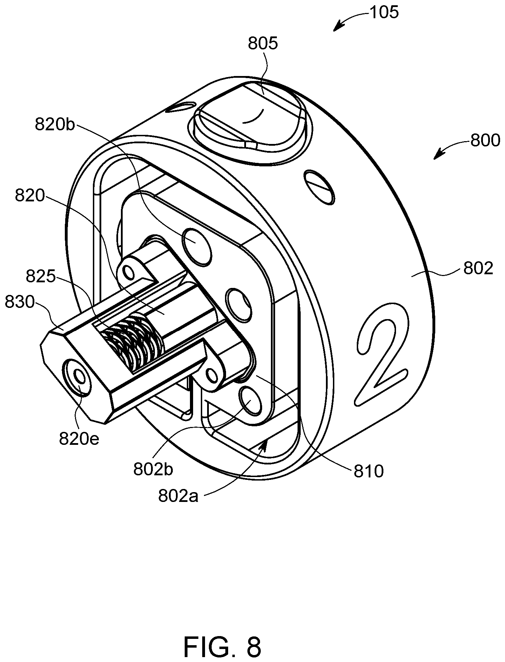

FIG. 8 is a perspective view of a control mechanism 800 of the handle assembly 105. The control mechanism 800 can include a puck 802 and an actuator 805 that can be used to move (proximally or distally) a suture catheter (e.g., suture catheter 420a, 420b, and/or 655). The puck 802 can be generally cylindrical and can define an interior space 802a to receive further components of the control mechanism 800, as will be explained in greater detail below. The actuator 805 can be a button that can be pressed and depressed to allow for movement of the puck 802 in a proximal and/or distal direction relative to handle assembly 105, causing the suture catheter to similarly be moved proximally and/or distally relative to other components of the catheter assembly 150 during loading, delivery, and/or deployment.

For example, and as explained above and depicted in FIG. 7, the suture catheter 655 can be advanced distally during loading to couple with a coupling ring (e.g., coupling ring 601) and retracted proximally to withdraw and collapse the prosthetic heart valve V into the loading funnel 650 and ultimately into the valve cover. As also explained above, during deployment, the suture catheter 655 can be advanced distally to disengage tethers from the prosthetic heart valve.

As shown in FIG. 8, the control mechanism 800 may include a bearing plate 810. The bearing plate 810 may be substantially square or rectangular in cross section and may have one or more rounded corners, allowing the bearing plate 810 to generally conform and fit at least partially or completely within the interior space 802a defined by the puck 802. However, this is just one example of a shape of the bearing plate 810, and other shapes may be suitable.

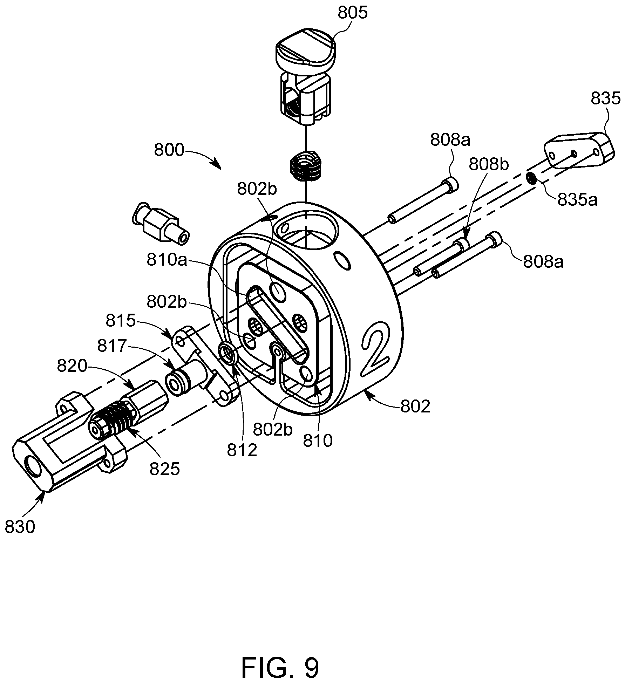

As best shown in Fig. 9, the bearing plate 810 may define a slot 810a in its distal face having a length direction extending substantially perpendicularly to a longitudinal axis of the handle assembly 105. The slot 810a may generally extend between opposing corners of the bearing plate 810. The slot 810a can be substantially stadium-shaped in cross section and may have one or more rounded corners, although other shapes, including rectangular, may be suitable. The slot 810a can have a depth such that the slot 810a (or at least a portion thereof) does not pass completely through the bearing plate 810. The size and shape of the slot 810a allows for a portion of a static piston 815 to be received within the slot 810a and thus by the bearing plate 810. The bearing plate 810 may include a plurality of through holes 802b, through which one or more alignment rods (not shown) can be passed to allow for guided movement of the puck 802 along the longitudinal axis of handle assembly 105. In some examples, a sealing component 812 (e.g., a gasket or polymer o-ring) can be provided between the static piston 815 and a floor of slot 810a.

The static piston 815 can have a base portion 815a that is substantially stadium-shaped (or otherwise shaped to be complementary to the shape of the slot 810a) and configured to be received by the slot 810a, and a substantially cylindrical portion 815b extending distally relative to the base portion 815a. The substantially cylindrical portion 815b can define a diameter such that it may be received within a translating bore nut 820.

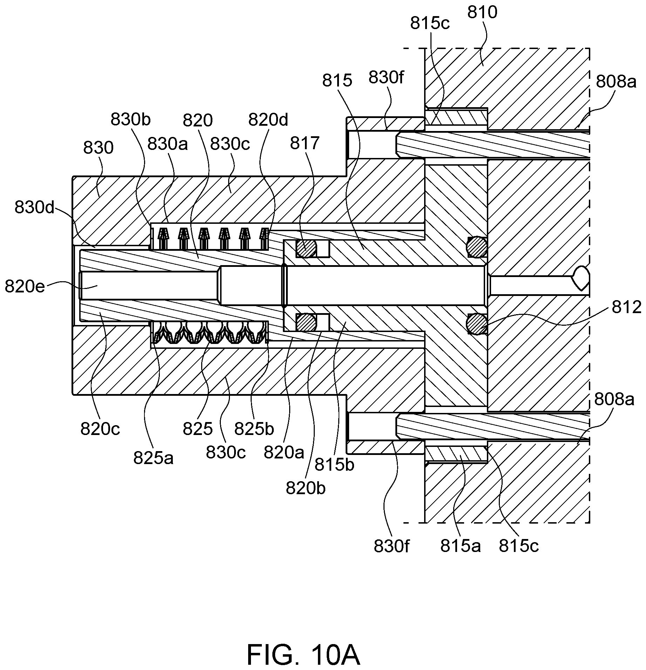

As shown in FIGS. 9 and 10A-B, one or more bolts 808a can be inserted into and through unthreaded openings 815c in static piston 815 and further inserted into openings 830f formed in spring bar 830 such that external threading on the distal ends of bolts 808a engages with the internal threading of openings 830f. In this regard, the static piston 815 is secured within slot 810a by virtue of being clamped between bearing plate 810 and spring bar 830.

At least one further bolt 808b can connect the puck 802 to bearing plate 810, for example by connecting the bearing plate 810 to an extension of the puck 802 received within a complementary-shaped recess within the bearing plate 810. A backing plate 835 can be positioned proximally relative to control mechanism 800 such that the backing plate 835 positions a sealing component 835a and allows a nosecone catheter to pass through the control mechanism 800.

The translating bore nut 820 can have a proximal portion 820a and a distal portion 820c. The proximal portion 820a can define an internal recess 820b that is substantially cylindrical in shape such that it can receive the substantially cylindrical portion 815b of static piston 815. A sealing component 817 (e.g., gasket or polymer o-ring) can be arranged circumferentially with respect to the substantially cylindrical portion (e.g., within an annular recess formed in the substantially cylindrical portion), such that the sealing component 817 is arranged (e.g. sandwiched) between the substantially cylindrical portion 815b and the inner wall of internal recess 820b.

The translating bore nut 820 is configured to translate proximally and/or distally relative to static piston 815. In this regard, a length of proximal portion 820a is larger than a total axis travel distance possible by translating bore nut 820. This ensures that no matter what translational position the translating bore nut 820 is in relative to static piston 815, the fluid seal is always maintained by seal 817 (i.e., the seal 817 is always sandwiched between the translating bore nut 820 and the static piston 815 no matter their relative translational displacement).

The translating bore nut 820 is configured to translate proximally and/or distally over the substantially cylindrical portion 815b, without having any (or any significant) lateral displacement of the translating bore nut 820. This is desirable, for example, because lateral displacement of the translating bore nut 820 relative to sealing component 817 could result in the loss of the fluid-tight seal between the inside of the translating bore nut 820 and the outside of the cylindrical portion 815b of the static piston 815. In some examples, this lateral displacement may be avoided, at least in part, by virtue of the guided movement of translating bore nut 820 over substantially cylindrical portion 815b and within the bore nut guide 830d of spring bar 830, described in greater detail below. This reduction in lateral displacement may also advantageously result in less axial resistance required to advance and retract catheters of the delivery assembly 150, including in particular the suture catheter.

A spring 825 can extend around an outer circumference of the distal portion 820c of the translating bore nut 820. A distal end 825a of the spring 825 may press upon an internal shoulder 830b defined by spring bar 830, while a proximal end 825b may press upon an external shoulder 820d defined at the transition between distal portion 820c and proximal portion 820a of translating bore nut 820. By virtue of the maximum distance between internal shoulder 830b and external shoulder 820d being smaller than the equilibrium length of spring 825, the spring 825 applies or exerts a load (e.g., spring restoring force) on spring bar 830 and/or translating bore nut 820 in all relative positions between the translating bore nut 820 and spring bar 830.

In the state depicted in FIG. 10A, the translating bore nut 820 is in its proximal-most translation state. In this state, the spring 825 exerts a minimum load on the translating bore nut 820, with the minimum load being greater than zero.

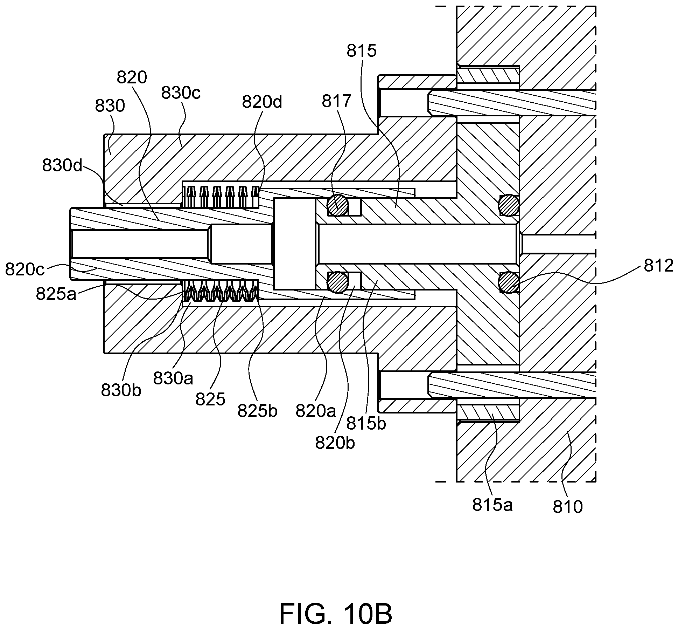

The spring bar 830 can define a partially bounded volume 830a between opposing legs 830c of the spring bar 830. The translating bore nut 820 and the spring 825 can be received within the partially bounded volume 830a between the opposing legs 830c. The spring bar 830 can also define a bore nut guide 830d for receiving at least a portion of the distal portion 820c of the translating bore nut 820. The bore nut guide 830d defines a diameter greater than a diameter of the translating bore nut 820 such that the translating bore nut 820 can translate proximally and distally relative to the spring bar 830, with distal movement of the translating bore nut 820 resulting in the distal portion 820c of the translating bore nut 820 extending through bore nut guide 830d and extending distally relative to a distal end of the spring bar 830 (as depicted in FIG 10B).

The translating bore nut 820 can also include a catheter connection interface 820e. The catheter connection interface 820e allows for a catheter, such as a suture catheter, to be coupled directly or indirectly to the translating bore nut 820.

As shown in FIG 10B, the translating bore nut 820 has translated distally from the state depicted in FIG. 10A. In this regard, the distance between internal shoulder 830b and external shoulder 820d has decreased and the distal portion 820c has extended distally beyond the spring bar 830 and bore nut guide 830d. In this state, the load exerted on translating bore nut 820 is greater than the load exerted in the state in FIG. 10A, resulting in an increased proximal bias on the suture catheter, and thus also on suture tethers coupled to the distal end of the suture catheter.

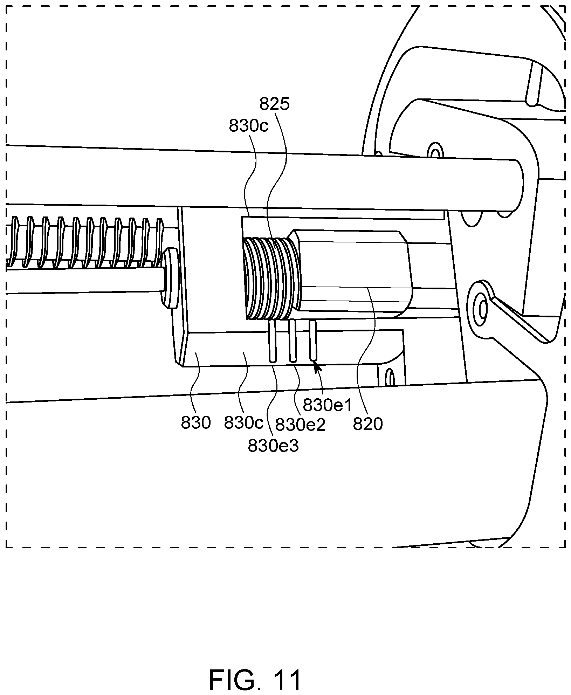

FIG. 11 is a perspective view of control mechanism 800 including tension indicator markings 830e1-3. The tension indicator markings 830e can be positioned on one or more legs 830c of the spring bar 830. The tension indicator markings 830e can align with a leading edge of translating bore nut 820 to indicate a level of tension currently present with respect to suture catheter and tethers.

The tension indicator markings 830e1-3 can be used during deployment such that alignment of a distal end of translating bore nut with respective markings is indicative of an amount of force exerted by the user beyond the minimum load applied by spring 825. On the other hand, the indicator markings 830e1-3 can be used during loading of the prosthetic heart valve into the funnel and/or valve cover.

For example, as the translating bore nut 820 is advanced distally from marking 830e1 to marking 830e2, the distance between internal shoulder 830b and external shoulder 820d decreases. Thus, the spring 825 compresses and the load applied by spring 825 increases to a second load greater than the minimum load. As the translating bore nut 820 is advanced distally from marking 830e2 to marking 830e3, the distance between internal shoulder 830b and external shoulder 820d further decreases. Thus, the spring 825 further compresses and the load applied by spring 825 increases to a third load greater than both the second load and the minimum load.

Axial translation of puck 802, can be monitored and controlled based upon visual feedback from the indicator markers 830e1-3 to ensure that the prosthetic heart valve is properly seated during loading and to verify that suture catheter and/or valve were not damaged during the loading process. For example, if translating bore nut 820 is advanced to the distal indicator 830e3 or distally beyond, it may be indicative that the tension on suture catheter and tethers is too great. On the other hand, if translating bore nut 820 retracts to the proximal indicator 830e1 or proximally beyond, it may be indicative that the tension on suture catheter and tethers is too low, indicating a possible risk of improper seating of the prosthetic heart valve into the valve cover and/or premature deployment of prosthetic heart valve.

The tension indicator markings 830e can be used for loading a prosthetic heart valve into the valve cover (e.g., valve cover 165) and can be used to set and sustain a tension level during loading of the valve. For example, tension indicator markings 830e can provide visual feedback to set and sustain the tension level by monitoring the position of translating bore nut 820 relative to one or more of the indicator markings 830e. By using tension indicator markings 830e, a calibrated torque driver may not be needed to load the prosthetic heart valve.

The tension indicator markings 830e can also be used prior to unsheathing of the prosthetic heart valve during delivery. In this regard, the tension indicator markings 830e can be used as a risk mitigation to verify that at least a minimum tension is maintained prior to unsheathing, as suture catheter tension may diminish during steering operations. The tension indicator markings 830e can be used to identify a first predetermined tension level (e.g., one of the markings 830e1-3 or a predetermined position between the markings) for use during loading of the valve. The tension indicator markings 830e can also be used to identify a second predetermined tension level (e.g., one of the markings 830e1-3 or a predetermined position between the markings), smaller than the first predetermined tension level, below which the tension should not drop prior to unsheathing to ensure proper seating of the prosthetic heart valve. The first and second predetermined tension levels can be identified and set based upon any number of factors, including diameter of the prosthetic heart valve (e.g., 28, 32, 36, or 40mm).

Further, the tension indicator markings 830e can be used to identify when tension is too great. In this regard, if the suture catheter tension is too high, it can become difficult to turn the steerable catheter during a steering operation.

In operation, as the catheter assembly is advanced through the vasculature and heart toward the native valve annulus, the catheter assembly may undergo one or more bends and/or turns. During such movements, the control mechanism 800 remains in an unactuated state. In this unactuated state, at least a force equal to or greater than the minimum load is applied to the translating bore nut 820 by the spring 825. This minimum load biases or urges the translating bore nut 820 proximally during the varying bends and/or turns of the catheter assembly, during which displacement between inner and outer catheters may occur. This minimum load thus prevents undesired axial translation of suture catheter and undesired slack in the tethers coupled to the prosthetic heart valve that would otherwise be caused by the displacement between inner and outer catheters. In other words, as the steering catheter is actuated to deflect the entire catheter assembly, in some situations the suture catheter may tend to translate distally relative to outer catheter which includes the valve cover that houses the prosthetic heart valve in the collapsed condition. If this particular relative translation were to occur, in the worst case the suture tethers of the suture catheter may slip off the prosthetic heart valve, resulting in premature disconnection between the prosthetic heart valve and the delivery system. In other cases, the suture tethers of the suture catheter may maintain their connection to the prosthetic heart valve, but slack may be introduced into the suture tethers, which could result in unexpected rapid movements of the prosthetic heart valve during deployment, as explained in greater detail below.

When deployment of the prosthetic heart valve is desired, a user may actuate valve cover retraction knob 115 to withdraw the valve cover relative to the prosthetic heart valve (and also relative to the valve retainer (e.g. valve retainer or "can" 590a or 590b) and relative to the suture catheter. As the valve cover retracts, the leading or ventricular end of the prosthetic heart valve may begin to self-expand for initial deployment and/or positioning. As this initial self-expansion occurs, the suture tethers help to ensure that the prosthetic heart valve does not prematurely fully deploy from the valve cover. In other words, one the leading end of the prosthetic heart valve begins to self-expand, expansion forces would tend to pull the prosthetic heart valve fully out of the valve cover. However, this is prevented as long as the suture tethers are connected to the prosthetic heart valve with appropriate tension. After this initial partial deployment, the user may confirm that the positioning is desirable (e.g. using imaging such as fluoroscopy) before deciding to fully deploy the prosthetic heart valve. In order to complete the deployment, the valve cover may be withdrawn further if desired and/or necessary, and the suture tethers may be disconnected from the prosthetic heart valve to achieve complete deployment and to disconnect the prosthetic heart valve from the delivery system. In order to disconnect the suture tethers from the prosthetic heart valve, the user may actuate the control mechanism 800, for example by depressing the button 805 and advancing the puck 802 distally relative to the handle assembly 105.

As noted above, the suture catheter may be coupled to the connection interface 820e such that as the puck 802 is translated along the housing 105a of the handle assembly 105, there is a corresponding translation of the suture catheter and the suture tethers coupled to the suture catheter. Thus, by advancing the puck 802 distally when it is time for completing deployment of the prosthetic heart valve, the suture catheter and suture tethers also advance distally, introducing slack into the suture tethers and allowing them to be pushed distally so that they disconnect from the prosthetic heart valve, as the suture tethers rely in part on being tensioned to maintain the connection to the prosthetic heart valve. Although the above description of movement of the puck 802 to advance or retract the suture catheter (and the suture tethers when they are coupled to the suture catheter), such movement of the suture catheter can also be implemented during loading, e.g., to couple the suture catheter to the coupling ring.

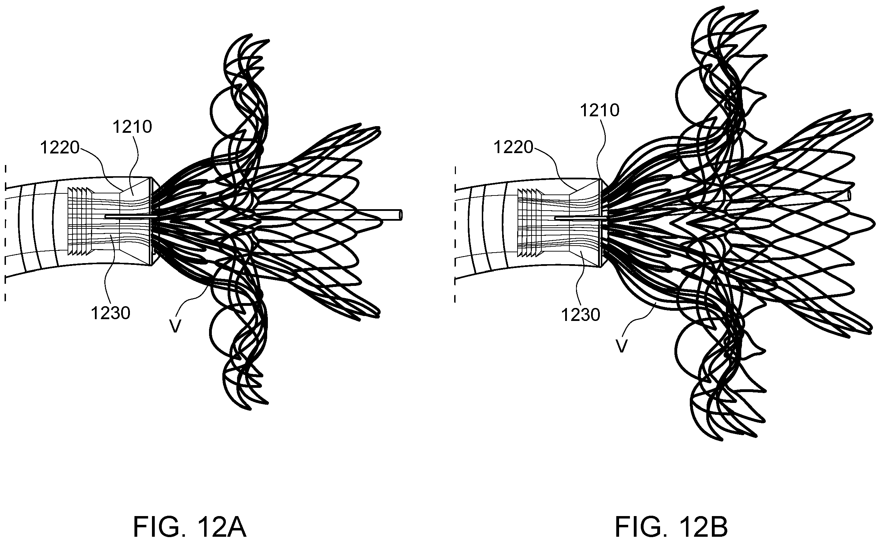

FIGS. 12A-B are fluoroscopic views of an initial stage of valve deployment, with FIG. 12A depicting initial valve deployment using a control mechanism with tensioning capabilities and FIG. 12B depicting initial valve deployment without tensioning capabilities. As shown in FIG. 12A, atrial petals 1210 (e.g. the trailing-most structure of an outer frame of the prosthetic heart valve) of the valve V are well contained within the valve retainer 1220. Further, radiopaque markers 1230 (e.g., radiopaque markers 640) on the tethers demonstrate good axial placement. This desirable positioning is achieved, at least in part, because the at least some threshold level of tension has been maintained on the suture tethers due to spring 825. Thus, as the leading-most or ventricular end of the prosthetic heart valve begins to self-expand, as shown in Fig. 12A, the tendency for the prosthetic heart valve to be pulled out of the valve cover under the prosthetic heart valve's own self-expansion forces is resisted by the counteracting forces provided by the tensioned suture tethers. On the other hand, FIG. 12B depicts atrial petals 1210 of the valve V having prematurely escaped (or on the verge of escaping) containment within valve retainer 1220. Further, radiopaque markers 1230 (e.g., radiopaque markers 640) on the tethers have migrated distally relative to their placement in FIG. 12A. This undesirable positioning resulted, at least in part, from the suture tethers having some amount of slack at the time that the leading or ventricular end of the prosthetic heart valve began to self-expand. In other words, the self-expansion forces of the leading or ventricular end of the prosthetic heart valve caused the prosthetic heart valve to begin to force itself distally out of the valve cover. This forward movement of the prosthetic heart valve was limited to the distance required for the slack in the suture tethers to be eliminated, at which point the suture tethers regained tension. While the regaining of the tension prevented complete premature deployment of the prosthetic heart valve (e.g. prevented the prosthetic heart valve from entirely deploying before the user was ready for such complete deployment), the prosthetic heart valve was effectively pulled forward relative to the delivery device such that the desirable positioning of the atrial petals 1210 within the valve retainer 1220 was lost, which may result in sub-optimal final deployment of the prosthetic heart valve when the user intentionally disconnects the suture tethers from the prosthetic heart valve.

Although the invention herein has been described with reference to particular embodiments, it is to be understood that these embodiments are merely illustrative of the principles and applications of the present invention.

## Claims

1. A system, comprising:
a delivery device (100) having a handle assembly (105) and a catheter assembly (105), the handle assembly having a control mechanism (800) with a spring (825), and the catheter assembly including a suture catheter (420a, 420b) operably coupled to the control mechanism such that axial translation of the control mechanism relative to the handle assembly correspondingly axially translates the suture catheter relative to the handle assembly;
a suture rigging assembly (600) having a plurality of suture tethers (615); and
a prosthetic heart valve (V),
wherein in an assembled condition of the system, the suture rigging assembly is coupled to the suture catheter, the plurality of suture tethers are coupled to the prosthetic heart valve, and the spring exerts a load to proximally bias the suture catheter relative to the control mechanism to maintain tension on the plurality of suture tethers.

2. The system of claim 1, wherein the control mechanism further comprises:
a translating bore nut (820) having a catheter connection interface (820e) that connects to the suture catheter, the translating bore nut having a distal portion (820c);
the spring extending around an outer circumference of the distal portion of the translating bore nut, a proximal end (825b) of the spring pressing upon an external shoulder (820d) of the translating bore nut and exerting the load.

3. The system of claim 2, wherein the control mechanism further comprises:
a spring bar (830) having an internal shoulder, a distal end (825a) of the spring pressing against the internal shoulder (830b) of the spring bar.

4. The system of claim 3, wherein the control mechanism further comprises:
a bearing plate (810) received within the control mechanism; and
a static piston (815) coupled to the bearing plate.

5. The system of claim 4, wherein the static piston includes at least one sealing component (812) arranged circumferentially with respect to a substantially cylindrical portion (815b) of the static piston such that a seal is maintained between the static piston and the translating bore nut.

6. The system of claim 5, wherein the translating bore nut translates axially relative to the static piston, where a length of a proximal portion (820a) of the translating bore nut is larger than a total axial travel distance of the translating bore nut such that the seal is maintained between the translating bore nut and the static piston.

7. The system of claim 3, wherein the spring bar defines a plurality of legs (830c) and a bore nut guide (830d), with a proximal portion of the translating bore nut being disposed between the plurality of legs and the distal portion of the translating bore nut being received within the bore nut guide.

8. The system of claim 3, wherein the spring bar includes a plurality of tension indicators (830e, 830e1-3) configured to align with a distal end of the translating bore nut in order to indicate a tension level of the plurality of suture tethers.

9. The system of claim 8, wherein the plurality of tension indicators are disposed on at least one leg of the spring bar.

10. The system of claim 3, wherein the bearing plate defines a longitudinal slot (810a) configured to receive a base portion (815a) of the static piston.

11. The system of claim 3, wherein the translating bore nut has a proximal portion (820a) defining an internal recess (820b) configured to receive a substantially cylindrical portion (815b) of the static piston.

12. The system of claim 1, wherein the control mechanism further comprises an actuator (805)_configured to allow for the axial translation of the control mechanism relative to the handle assembly.

13. The system of claim 12, wherein the suture catheter is configured to be advanced distally to engage with a coupling ring (601) of the suture rigging assembly.

14. The system of claim 13, wherein, upon coupling of the suture catheter and coupling ring, the suture catheter is retracted proximally to load the prosthetic heart valve in a valve cover (165) of the catheter assembly.

15. The system of claim 13, wherein in the assembled condition of the system, the suture catheter is configured to be advanced distally by distally translating the control mechanism relative to the handle assembly to disconnect the plurality of suture tethers from the prosthetic heart valve to allow for deployment of the prosthetic heart valve into a native valve.
